# EUROPÄISCHE PATENTSCHRIFT

(11) **EP 1 656 143 B1**
(45) Veröffentlichungstag und Bekanntmachung des Hinweises auf die Patenterteilung: **12.10.2011**
(21) Anmeldenummer: 04763700.4
(22) Anmeldetag: 02.08.2004
(51) Int. Cl.: A61K 31/4709, A61K 31/4745, A61P 31/04

(54) **CHINOLON-ANTIBIOTIKA ZUR BEHANDLUNG VON PERIODONTALER INFEKTIONEN**
NOVEL USE OF QUINOLONE ANTIBIOTICS
NOUVELLE UTILISATION D'ANTIBIOTIQUES DU GROUPE QUINOLONE

(30) Priorität: 13.08.2003 DE 10337191
(43) Veröffentlichungstag der Anmeldung: 17.05.2006
(73) Patentinhaber: Bayer Animal Health GmbH, 51368 Leverkusen (DE)
(72) Erfinder: DAUBE, Gert, 51766 Engelskirchen (DE); EDINGLOH, Markus, 51379 Leverkusen (DE); STEPHAN, Bernd, 40789 Monheim (DE); PIRRO, Franz, 40764 Langenfeld (DE); LIMET, Agnès, F-92100 Boulogne (FR)
(86) Internationale Anmeldenummer: PCT/EP2004/008629
(87) Internationale Veröffentlichungsnummer: WO 2005/018641

(56) Entgegenhaltungen:
- WO-A-97/31001
- "Research workshop on moxifloxacin" CHEMOTHERAPIE JOURNAL, SUPPLEMENT 2003 GERMANY, Bd. 12, Nr. 23, Februar 2003 (2003-02), Seiten 1-8, XP009039149 ISSN: 0944-6486
- KLEINFELDER J W ET AL: "Fluoroquinolones in the treatment of Actinobacillus actinomycetemcomitans-associated periodontitis." JOURNAL OF PERIODONTOLOGY. FEB 2000, Bd. 71, Nr. 2, Februar 2000 (2000-02), Seiten 202-208, XP009039157 ISSN: 0022-3492
- SASAKI J: "Clinical studies of sparfloxacin in odontogenic infections" DRUGS 1993 NEW ZEALAND, Bd. 46, Nr. SUPPL. 3, 1993, Seite 331, XP009039162 ISSN: 0012-6667
- SLOTS JORGEN: "Selection of antimicrobial agents in periodontal therapy" JOURNAL OF PERIODONTAL RESEARCH, Bd. 37, Nr. 5, Oktober 2002 (2002-10), Seiten 389-398, XP009039156 ISSN: 0022-3484
- FRAATZ K ET AL: "Skin concentrations and serum pharmacokinetics of pradofloxacin in dogs after multiple oral administrations at four different dosages." JOURNAL OF VETERINARY PHARMACOLOGY AND THERAPEUTICS, Bd. 26, Nr. Supplement 1, August 2003 (2003-08), Seite 89, XP002303797 & PROCEEDINGS OF THE 9TH INTERNATIONAL CONGRESS OF THE EUROPEAN ASSOCIATION FOR VETERINARY PHARMACOLOG; LISBON, PORTUGAL; JULY 13-18, 2003 ISSN: 0140-7783
- DATABASE BIOSIS [Online] BIOSCIENCES INFORMATION SERVICE, PHILADELPHIA, PA, US; 2002, ABRAHAM J ET AL: "Pradofloxacin: Comparative in vitro activity against selected pathogens from the US." XP002303798 Database accession no. PREV200300265605 & ABSTRACTS OF THE INTERSCIENCE CONFERENCE ON ANTIMICROBIAL AGENTS AND CHEMOTHERAPY, Bd. 42, 2002, Seite 189, AMERICAN SOCIETY FOR MICROBIOLOGY (ASM) ANNUAL MEETING ON INFECTIOUS DISAEASE; SAN DIEGO, CA, USA; SEPTEMBER 27-30, 2002
- DATABASE BIOSIS [Online] BIOSCIENCES INFORMATION SERVICE, PHILADELPHIA, PA, US; Januar 2002 (2002-01), WISE R ET AL: "Single-dose pharmacokinetics and penetration of BMS 284756 into an inflammatory exudate" XP002337675 Database accession no. PREV200200068707 & ANTIMICROBIAL AGENTS AND CHEMOTHERAPY, Bd. 46, Nr. 1, Januar 2002 (2002-01), Seiten 242-244, ISSN: 0066-4804
- DATABASE BIOSIS [Online] BIOSCIENCES INFORMATION SERVICE, PHILADELPHIA, PA, US; März 2002 (2002-03), GOLDSTEIN ELLIE J C ET AL: "In vitro activities of the des-fluoro(6) quinolone BMS-284756 against aerobic and anaerobic pathogens isolated from skin and soft tissue animal and human bite wound infections" XP002337676 Database accession no. PREV200200187376 & ANTIMICROBIAL AGENTS AND CHEMOTHERAPY, Bd. 46, Nr. 3, März 2002 (2002-03), Seiten 866-870, ISSN: 0066-4804
- HECHT D W ET AL: "ACTIVITIES OF GARENOXACIN (BMS-284756) AND OTHER AGENTS AGAINST ANAEROBIC CLINICAL ISOLATES" ANTIMICROBIAL AGENTS AND CHEMOTHERAPY, AMERICAN SOCIETY FOR MICROBIOLOGY, WASHINGTON, DC, US, Bd. 47, Nr. 3, März 2003 (2003-03), Seiten 910-916, XP001203532 ISSN: 0066-4804
- GOLDSTEIN ELLIE J C ET AL: "In vitro activities of a new des-fluoroquinolone, BMS 284756, and seven other antimicrobial agents against 151 isolates of Eikenella corrodens" ANTIMICROBIAL AGENTS AND CHEMOTHERAPY, Bd. 46, Nr. 4, April 2002 (2002-04), Seiten 1141-1143, XP002337663 ISSN: 0066-4804
- DATABASE EMBASE [Online] ELSEVIER SCIENCE PUBLISHERS, AMSTERDAM, NL; 1996, DUBREUIL L ET AL: "In vitro activity of a new fluoroquinolone, marbofloxacin (RO 09-1168), against anaerobes and some bacteria from the human gut" XP002337677 Database accession no. EMB-1996193705 & PATHOLOGIE BIOLOGIE 1996 FRANCE, Bd. 44, Nr. 5, 1996, Seiten 333-336, ISSN: 0369-8114
- DATABASE BIOSIS [Online] BIOSCIENCES INFORMATION SERVICE, PHILADELPHIA, PA, US; 1996, SCHNEIDER M ET AL: "Pharmacokinetics of marbofloxacin in dogs after oral and parenteral administration" XP002337678 Database accession no. PREV199698772498 & JOURNAL OF VETERINARY PHARMACOLOGY AND THERAPEUTICS, Bd. 19, Nr. 1, 1996, Seiten 56-61, ISSN: 0140-7783
- DATABASE EMBASE [Online] ELSEVIER SCIENCE PUBLISHERS, AMSTERDAM, NL; 2003, DA SILVA A D ET AL: "Biological activity and synthetic metodologies for the preparation fluoroquinoles, a class of potent antibacterial agents" XP002337679 Database accession no. EMB-2003047644 & CURRENT MEDICINAL CHEMISTRY 2003 NETHERLANDS, Bd. 10, Nr. 1, 2003, Seiten 21-39, ISSN: 0929-8673
- DATABASE BIOSIS [Online] BIOSCIENCES INFORMATION SERVICE, PHILADELPHIA, PA, US; April 2002 (2002-04), COULET M ET AL: "Pharmacokinetics of ibafloxacin following intravenous and oral administration to healthy beagle dogs" XP002337680 Database accession no. PREV200200317403 & JOURNAL OF VETERINARY PHARMACOLOGY AND THERAPEUTICS, Bd. 25, Nr. 2, April 2002 (2002-04), Seiten 89-97, ISSN: 0140-7783
- DATABASE BIOSIS [Online] BIOSCIENCES INFORMATION SERVICE, PHILADELPHIA, PA, US; 1993, WATTS J L ET AL: "In vivo activity of U-95376A (PD-140288), a new fluoroquinolone, against pathogens of veterinary importance" XP002337681 Database accession no. PREV199497440213 & PROGRAM AND ABSTRACTS OF THE INTERSCIENCE CONFERENCE ON ANTIMICROBIAL AGENTS AND CHEMOTHERAPY, Bd. 33, Nr. 0, 1993, Seite 338, 33RD INTERSCIENCE CONFERENCE ON ANTIMICROBIAL AGENTS AND CHEMOTHERAPY; NEW ORLEANS, LOUISIANA, USA; OCTOBER 17-20, 1993 ISSN: 0733-6373
- MCGUIRK P R ET AL: "SYNTHESIS AND STRUCTURE-ACTIVITY RELATIONSHIPS OF 7-DIAZABICYCLOALKYLQUINOLONES INCLUDING DANOFLOXACIN A NEW QUINOLONE ANTIBACTERIAL AGENT FOR VETERINARY MEDICINE" JOURNAL OF MEDICINAL CHEMISTRY, AMERICAN CHEMICAL SOCIETY. WASHINGTON, US, Bd. 35, Nr. 4, 21. Februar 1992 (1992-02-21), Seiten 611-620, XP002901194 ISSN: 0022-2623
- DATABASE BIOSIS [Online] BIOSCIENCES INFORMATION SERVICE, PHILADELPHIA, PA, US; 1987, BANSAL M B ET AL: "ACTIVITY OF DIFLOXACIN A-56619 AND A-56620 AGAINST CLINICAL ANAEROBIC BACTERIA IN-VITRO" XP002337682 Database accession no. PREV198784001740 & ANTIMICROBIAL AGENTS AND CHEMOTHERAPY, Bd. 31, Nr. 4, 1987, Seiten 619-621, ISSN: 0066-4804
- DATABASE EMBASE [Online] ELSEVIER SCIENCE PUBLISHERS, AMSTERDAM, NL; 1986, GOLDSTEIN E J C ET AL: "Susceptibility of Eikenella corrodens to newer and older quinolones" XP002337683 Database accession no. EMB-1986176457 & ANTIMICROBIAL AGENTS AND CHEMOTHERAPY 1986 UNITED STATES, Bd. 30, Nr. 1, 1986, Seiten 172-173,
- DATABASE BIOSIS [Online] BIOSCIENCES INFORMATION SERVICE, PHILADELPHIA, PA, US; 1986, GRANNEMAN G R ET AL: "DIFLOXACIN METABOLISM AND PHARMACOKINETICS IN HUMANS AFTER SINGLE ORAL DOSES" XP002337684 Database accession no. PREV198783031439 & ANTIMICROBIAL AGENTS AND CHEMOTHERAPY, Bd. 30, Nr. 5, 1986, Seiten 689-693, ISSN: 0066-4804
- DATABASE MEDLINE [Online] US NATIONAL LIBRARY OF MEDICINE (NLM), BETHESDA, MD, US; Januar 2002 (2002-01), KAY-MUGFORD PATRICIA A ET AL: "Determination of plasma and skin concentrations of orbifloxacin in dogs with clinically normal skin and dogs with pyoderma." XP002337685 Database accession no. NLM12584677 & VETERINARY THERAPEUTICS : RESEARCH IN APPLIED VETERINARY MEDICINE. WINTER 2002, Bd. 3, Nr. 4, Januar 2002 (2002-01), Seiten 402-408, ISSN: 1528-3593
- LU ET AL: 'Enhancement of Fluoroquinolone Activity by C-8 Halogen and Methoxy Moieties: Action against a Gyrase Resistance Mutant of Mycobacterium smegmatis and a Gyrase-Topoisomerase IV Double Mutant of Staphylococcus aureus' ANTIMICROBIAL AGENTS AND CHEMOTHERAPY Bd. 45, Nr. 10, Oktober 2001, Seiten 2703 - 2709
- CIANCHETTA ET AL.: 'Chemometric Studies on the Bactericidal Activity of Quinolones via an Extended VolSurf Approach' J.MED.CHEM. Bd. 47, 05 Juli 2004, Seiten 3193 - 3201
- LU ET AL: "Enhancement fo Fluoroquinolone activity..." ANTIMICROBIAL AGENTS AND CHEMOTHERAPY, vol. 45, no. 10, October 2001 (2001-10), pages 2703-2709,
- CIANCHETTA ET AL.: "Chemometric strudies on the bactericidal activity of quinolones..." J.MED.CHEM., vol. 47, 5 July 2004 (2004-07-05), pages 3193-3201,

## Beschreibung

Die vorliegende Erfindung betrifft die Verwendung von bestimmten Chinolon-Antibiotika zur Bekämpfung von bakteriellen Erkrankungen der Mundhöhle, insbesondere in der Tiermedizin.

Für verschiedene Fluorchinolon-Antibiotika wurde bereits eine *in vitro* Wirkung gegen anaerobe Bakterien beschrieben, darunter auch solche, die üblicherweise bei Infektionen in der Mundhöhle auftreten (Siehe: Sobotka I et al.: In vitro activity of moxifloxacin against bacteria isolated from odontogenic abscesses. Antimicrobial Agents and Chemotherapy 46, 4019-4021, 2002; Muller HP et al.: In vitro antimicrobial susceptibility of oral strains of Actinobacillus actinomycetemcomitans to seven antibiotics. Journal of Clinical Periodontology 29, 736-742, 2002; Pfister W et al.: Activity of quinolones against anaerobic and capnophilic bacteria in vitro. Deutsche Zahnärztliche Zeitschrift 56, 189-192, 2001; Goldstein EJC et al.: In vitro activities of a new desfluoroquinolone, BMS 284756, and seven other antimicrobial agents against 151 isolates of Eikenella corrodens. Antimicrobial Agents and Chemotherapy 46, 1141-1143, 2002; Hecht DW, Osmolski JR (2003): Activities of garenoxacin (BMS-284756) and other agents against anaerobic clinical isolates. Antimicrobial Agents and Chemotherapy 47, 910-916).

Weiterhin wurde in WO 01/45679 beschrieben, dass Fluorchinolon-Antibiotika sich bei lokaler bzw. topischer Anwendung zur Behandlung von bakteriellen Erkrankungen insbesondere in der Mundhöhle eignen, beispielsweise bei der Wurzelbehandlung in der Zahnmedizin.

Weiterhin wurde Ciprofloxacin in der Therapie der Parodontitis (= Periodontal Disease) eingesetzt. Es wird aber eine Kombinationstherapie mit Metronidazol empfohlen (Siehe: Mombelli AW, van Winkelhoff AJ: The systemic use of antibiotics in periodontal therapy. In: Proceedings of the 2nd European Workshop on Periodontology. Chemicals in Periodontics. Eds.: Lang NP, Karring T, Lindhe J. Thurgau, Switzerland, 1996. Quintessenz Books, Berlin, 38-77).

Auch die Kombination von Enrofloxacin und Metronidazol zur Therapie von Parodontitis bei Hunden und Katzen wurde beschrieben (siehe: Nielsen D: Clinical Experience with an Enrofloxacin-Metronidazole Combination in the Treatment of Periodontal Disease in Dogs and Cats. In: Proceedings of the Third International Veterinary Symposium on Baytril. Ed. Ford RB. Seville, Spain, 1999, 88-94).

Pradofloxacin und seine antibiotische Wirkung sind in WO97/31001 beschrieben.

Nach wie vor besteht jedoch Bedarf an Wirkstoffen, die sich bei systemischer Applikation allein oder in Kombination mit anderen Antibiotika oder Chemotherapeutika zur Behandlung von bakteriellen Erkrankungen der Mundhöhle eignen. Dabei ist eine möglichst hohe Aktivität und ein möglichst breites Wirkspektrum gegen die bei Mundhöhlenerkrankungen typischen anaeroben Bakterien wünschenswert. Vorzugsweise könnte so auf die übliche Kombinationstherapie verzichtet werden, da die Monotherapie mit nur einem Wirkstoff ausreicht.

Die Erfindung betrifft daher die Verwendung von

8-Cyano-Chinolon-Antibiotika

zur Herstellung von Arzneimitteln zur systemischen Behandlung von bakteriellen Erkrankungen der Mundhöhle.

8-Cyano-chinolone sind insbesondere die der Formel (I): 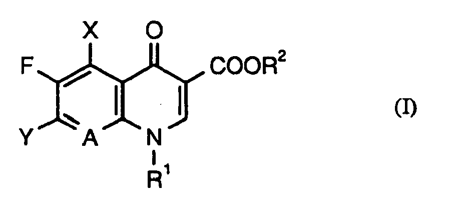 in welcher
- X: für Wasserstoff, Halogen, C₁₋₄-Alkyl, C₁₋₄-Alkoxy, NH₂ steht,
- Y: für Reste der Strukturen

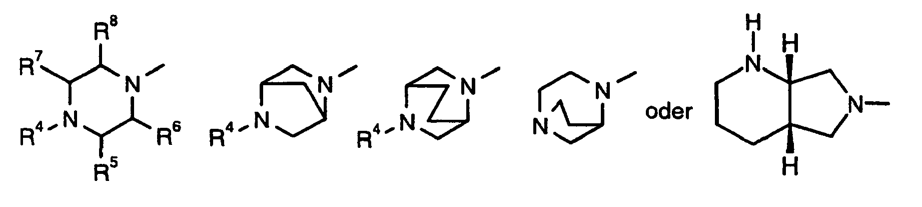

- steht, worin R⁴: für gegebenenfalls durch Hydroxy oder Methoxy substituiertes geradkettiges oder verzweigtes C₁-C₄-Alkyl, Cyclopropyl, Acyl mit 1 bis 3 C-Atomen steht,
- R⁵: für Wasserstoff, Methyl, Phenyl, Thienyl oder Pyridyl steht,
- R⁶: für Wasserstoff oder C₁₋₄-Alkyl steht,
- R⁷: für Wasserstoff oder C₁₋₄-Alkyl steht,
- R⁸: für Wasserstoff oder C₁₋₄-Alkyl steht,
sowie
- R¹: für einen Alkylrest mit 1 bis 3 Kohlenstoffatomen, Cyclopropyl, 2-Fluorethyl, Methoxy, 4-Fluorphenyl, 2,4-Difluorphenyl oder Methylamino steht,
- R²: für Wasserstoff oder gegebenenfalls durch Methoxy oder 2-Methoxyethoxy substituiertes Alkyl mit 1 bis 6 Kohlenstoffatomen sowie Cyclohexyl, Benzyl, 2-Oxopropyl, Phenacyl, Ethoxycarbonylmethyl, Pivaloyloxymethyl steht,
- A: für =C(CN) steht,
und deren pharmazeutisch verwendbare Salze und Hydrate.

Bevorzugt sind Verbindungen der Formel (I),
in welcher
- A: für =C-CN steht,
- R¹: für gegebenenfalls durch Halogen substituiertes C₁-C₃-Alkyl oder Cyclopropyl steht,
- R²: für Wasserstoff oder C₁₋₄-Alkyl steht,
- Y: für Reste der Strukturen

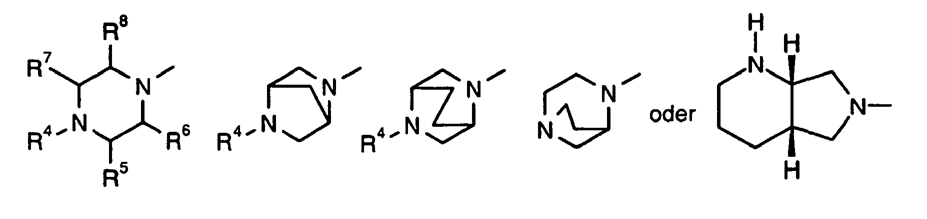

- steht, worin R⁴: für gegebenenfalls durch Hydroxy substituiertes geradkettiges oder verzweigtes C ₁-C₃-Alkyl, Oxalkyl mit 1 bis 4 C-Atomen steht,
- R⁵: für Wasserstoff, Methyl oder Phenyl steht,
- R⁷: für Wasserstoff oder Methyl steht,
- R⁶ und R⁸: für Wasserstoff stehen,
und deren pharmazeutisch verwendbare Hydrate und Salze.

Besonders bevorzugt sind Verbindungen der Formel (I),
in welcher
- A: für =C-CN steht,
- R¹: für Cyclopropyl steht,
- R²: für Wasserstoff, Methyl oder Ethyl steht,
- Y: für Reste der Strukturen 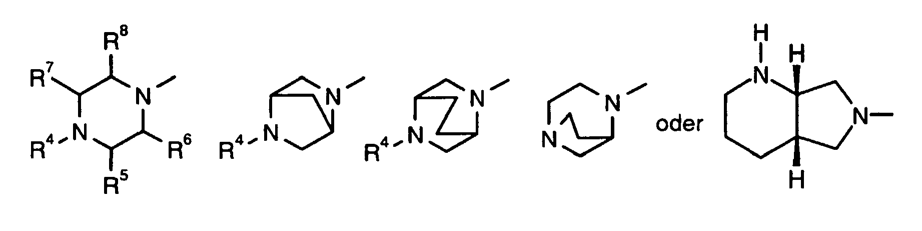
steht, worin
- R⁴: für Methyl, gegebenenfalls durch Hydroxy substituiertes Ethyl steht,
- R⁵: für Wasserstoff oder Methyl steht,
- R⁷: für Wasserstoff oder Methyl steht,
- R⁶ und R⁸: für Wasserstoff stehen,
und deren pharmazeutisch verwendbare Salze und Hydrate.

Als besonders bevorzugtes 8-Cyano-Chinolon sei Pradofloxacin genannt, es hat die systematische Bezeichnung 8-Cyano-1-cyclopropyl-7-((1S,6S)-2,8-diazabicyclo[4.3.0]nonan-8-yl)-6-fluor-1,4-dihydro-4-oxo-3-chinotincarbonsäure und die Formel

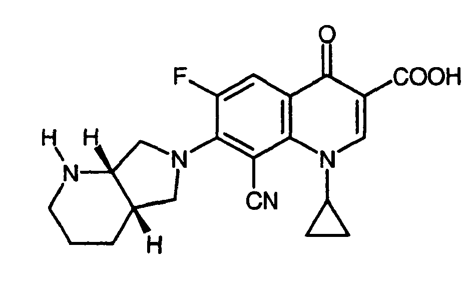

Im Sinne dieser Erfindung kann Pradofloxacin in Form seiner pharmazeutisch verträglichen Prodrugs und Salze eingesetzt werden. Ebenfalls eingeschlossen sind Hydrate und andere Solvate dieser Verbindungen. Geeignete Prodrugs und Salze sind z.B. in WO 97/31001 beschrieben, auf dieses Dokument wird hiermit ausdrücklich Bezug genommen. Es wurde gefunden, dass Pradofloxacin ein breites antibakterielles Wirkspektrum hat, insbesondere gegen Keime, die bei Mundhöhleninfektionen eine Rolle spielen. Pradofloxacin eignet sich daher besonders für die Monotherapie von bakteriellen Erkrankungen der Mundhöhle.

Als Salze kommen pharmazeutisch verwendbare Säureadditionssalze und basische Salze in Frage.

Als pharmazeutisch verwendbare Salze sind beispielsweise die Salze der Salzsäure, Schwefelsäure, Essigsäure, Glykolsäure, Milchsäure, Bernsteinsäure, Zitronensäure, Weinsäure, Methansulfonsäure, 4-Toluolsulfonsäure, Galacturonsäure, Gluconsäure, Embonsäure, Glutaminsäure oder Asparaginsäure zu verstehen. Ferner lassen sich die erfindungsgemäßen Verbindungen an saure oder basische Ionenaustauscher binden. Als pharmazeutisch verwendbare basische Salze seien die Alkalisalze, beispielsweise die Natrium- oder Kaliumsalze, die Erdalkalisalze, beispielsweise die Magnesium-, oder Calciumsalze; die Zinksalze, die Silbersalze und die Guanidiniumsalze genannt.

Unter Hydraten werden sowohl die Hydrate der Fluorchinolone selbst als auch die Hydrate von deren Salzen verstanden.

Erfindungsgemäß können sowohl die Enantiomere als auch Enantiomerengemische der genannten Chinolone eingesetzt werden.

Die Herstellung der Chinolone ist bekannt (siehe z.B. WO 97/31001 für die Herstellung von Pradofloxacin) oder kann analog zu bekannten Verfahren erfolgen.

Durch Bakterien verursachte Mundhöhlenerkrankungen sind insbesondere: Gingivitis, Parodontitis, Stomatitis und orale Abszesse.

Bei diesen Erkrankungen spielen vor allem anaerobe Bakterien eine Rolle, insbesondere: *Porphyromonas* spp., *Prevotella* spp., *Bacteroides* spp., *Actinobacillus actinomycetemcomitans, Fusobacterium* spp., *Peptostreptococcus* spp., *Eikenella corrodens, Capnocytophaga ochracea* und *Campylobacter rectus.* Erfindungsgemäß wird eine gute Wirkung gegen die entsprechenden anaeroben Bakterien erreicht.

Mit den vorstehend genannten Chinolonen können bakterielle Mundhöhlenerkrankungen mittels systemischer Applikation bei Menschen und Tieren (Nutz-, Zoo-, Labor-, Versuchs- und Hobbytieren) behandelt werden.

Zu den Nutztieren gehören Säugetiere wie z.B. Rinder, Pferde, Schafe, Schweine, Ziegen, Kamele, Wasserbüffel, Esel, Kaninchen, Damwild, Rentiere, Pelztiere wie z.B. Nerze, Chinchilla, Waschbär; Vögel wie z.B. Hühner, Gänse, Puten, Enten, Tauben, Strauße, Vogelarten für Heim- und Zoohaltung. Ferner gehören dazu Nutz- und Zierfische.

Zu Labor- und Versuchstieren gehören Mäuse, Ratten, Meerschweinchen, Goldhamster, Hunde und Katzen.

Zu den Hobbytieren gehören Hunde und Katzen.

Bevorzugt werden Säugetiere behandelt, insbesondere Hunde oder Katzen.

Die Anwendung kann sowohl prophylaktisch als auch therapeutisch erfolgen.

Aufgrund des breiten Wirkspektrums der genannten Chinolone - auch gegen anaerobe Bakterien - eignen sie sich insbesondere für die Monotherapie.

Die Anwendung der Wirkstoffe erfolgt direkt oder in Form von geeigneten Zubereitungen enteral oder parenteral.

Die enterale Anwendung der Wirkstoffe geschieht z.B. oral in Form von Pulver, Zäpfchen, Tabletten, Kapseln, Pasten, Tränken, Granulaten, Drenchen, Boli, medikiertem Futter oder Trinkwasser. Die parenterale Anwendung geschieht z.B. in Form der Injektion (intramusculär, subcutan, intravenös, intraperitoneal) oder durch Implantate.

Geeignete Zubereitungen sind:
Lösungen wie Injektionslösungen, orale Lösungen, Konzentrate zur oralen Verabreichung nach Verdünnung;
Emulsionen und Suspension zur oralen Anwendung sowie zur Injektion; Halbfeste Zubereitungen;
Formulierungen, bei denen der Wirkstoff in einer Öl in Wasser oder Wasser in Öl Emulsionsgrundlage verarbeitet ist;
Feste Zubereitungen wie Pulver, Premixe oder Konzentrate, Granulate, Pellets, Tabletten, Boli, Kapseln; Aerosole und Inhalate, wirkstoffhaltige Formkörper.
Injektionslösungen werden intravenös, intramuskulär oder subcutan verabreicht.

Injektionslösungen werden hergestellt, indem der Wirkstoff in einem geeigneten Lösungsmittel gelöst wird und eventuell Zusätze wie Lösungsvermittler, Säuren, Basen, Puffersalze, Antioxidantien, Konservierungsmittel zugefügt werden. Die Lösungen werden steril filtriert und abgefüllt.

Als Lösungsmittel seien genannt: Physiologisch verträgliche Lösungsmittel wie Wasser; Alkohole wie Ethanol, Butanol, Benzylalkohol, Glycerin; Kohlenwasserstoffe, Propylenglykol, Polyethylenglykole, N-Methylpyrrolidon, sowie Gemische derselben.

Die Wirkstoffe lassen sich gegebenenfalls auch in physiologisch verträglichen pflanzlichen oder synthetischen Ölen, die zur Injektion geeignet sind, lösen.

Als Lösungsvermittler seien genannt: Lösungsmittel, die die Lösung des Wirkstoffs im Hauptlösungsmittel fördern oder sein Ausfallen verhindern. Beispiele sind Polyvinylpyrrolidon, polyoxyethyliertes Rhizinusöl, polyoxyethylierte Sorbitanester.

Konservierungsmittel sind: Benzylalkohol, Trichlorbutanol, p-Hydroxybenzoesäureester, n-Butanol.

Orale Lösungen werden direkt angewendet. Konzentrate werden nach vorheriger Verdünnung auf die Anwendungskonzentration oral angewendet. Orale Lösungen und Konzentrate werden, wie oben bei den Injektionslösungen beschrieben, hergestellt, wobei auf steriles Arbeiten verzichtet werden kann.

Es kann vorteilhaft sein, bei der Herstellung Verdickungsmittel zuzufügen. Verdickungsmittel sind: Anorganische Verdickungsmittel wie Bentonite, kolloidale Kieselsäure, Aluminiummonostearat, organische Verdickungsmittel wie Cellulosederivate, Polyvinylalkohole und deren Copolymere, Acrylate und Metacrylate.

Als Lösungsmittel seien genannt: Wasser, Alkanole, Glycole, Polyethylenglycole, Polypropylenglycole, Glycerin, aromatische Alkohole wie Benzylalkohol, Phenylethanol, Phenoxyethanol, Ester wie Essigester, Butylacetat, Benzylbenzoat, Ether wie Alkylenglykolalkylether wie Dipropylenglykolmonomethylether, Diethylenglykolmono-butylether, Ketone wie Aceton, Methylethylketon, aromatische und/oder aliphatische Kohlenwasserstoffe, pflanzliche oder synthetische Öle, DMF, Dimethylacetamid, N-Methylpyrrolidon, 2-Dimethyl-4-oxy-methylen-1,3-dioxolan.

Farbstoffe sind alle zur Anwendung am Tier zugelassenen Farbstoffe, die gelöst oder suspendiert sein können.

Antioxidantien sind Sulfite oder Metabisulfite wie Kaliummetabisulfit, Ascorbinsäure, Butylhydroxytoluol, Butylhydroxyanisol, Tocopherol.

Emulsionen können oral oder als Injektionen angewendet werden.

Emulsionen sind entweder vom Typ Wasser in Öl oder vom Typ Öl in Wasser.

Sie werden hergestellt, indem man den Wirkstoff entweder in der hydrophoben oder in der hydrophilen Phase löst und diese unter Zuhilfenahme geeigneter Emulgatoren und gegebenenfalls weiterer Hilfsstoffe wie Farbstoffe, resorptionsfördernde Stoffe, Konservierungsstoffe, Antioxidantien, Lichtschutzmittel, viskositätserhöhende Stoffe, mit dem Lösungsmittel der anderen Phase homogenisiert.

Als hydrophobe Phase (Öle) seien genannt: Paraffinöle, Silikonöle, natürliche Pflanzenöle wie Sesamöl, Mandelöl, Rizinusöl, synthetische Triglyceride wie Capryl/Caprinsäure-biglycerid, Triglyceridgemisch mit Pflanzenfettsäuren der Kettenlänge C₈₋₁₂ oder anderen speziell ausgewählten natürlichen Fettsäuren, Partialglyceridgemische gesättigter oder ungesättigter, eventuell auch hydroxylgruppenhaltiger Fettsäuren, Mono- und Diglyceride der C₈/C₁₀-Fettsäuren.

Fettsäureester wie Ethylstearat, Di-n-butyryl-adipat, Laurinsäurehexylester, Dipropylen-glykolpelargonat, Ester einer verzweigten Fettsäure mittlerer Kettenlänge mit gesättigten Fettalkoholen der Kettenlänge C₁₆-C₁₈, Isopropylmyristat, Isopropylpalmitat, Capryl/Caprinsäureester von gesättigten Fettalkoholen der Kettenlänge C₁₂-C₁₈, Isopropylstearat, Ölsäureoleylester, Ölsäuredecylester, Ethyloleat, Milchsäureethylester, wachsartige Fettsäureester wie Dibutylphthalat, Adipinsäurediisopropylester, letzterem verwandte Estergemische u.a. Fettalkohole wie Isotridecylalkohol, 2-Octyldodecanol, Cetylstearyl-alkohol, Oleylalkohol.

Fettsäuren wie z.B. Ölsäure und ihre Gemische.

Als hydrophile Phase seien genannt:

Wasser, Alkohole wie z.B. Propylenglycol, Glycerin, Sorbitol und ihre Gemische.

Als Emulgatoren seien genannt:
nichtionogene Tenside, z.B. polyoxyethyliertes Rizinusöl, polyoxyethyliertes Sorbitan-monooleat, Sorbitanmonostearat, Glycerinmonostearat, Polyoxyethylstearat, Alkylphenolpolyglykolether;
ampholytische Tenside wie Di-Na-N-lauryl-ß-iminodipropionat oder Lecithin;
anionaktive Tenside, wie Na-Laurylsulfat, Fettalkoholethersulfate, Mono/Dialkylpolyglykoletherorthophosphorsäureester-monoethanolaminsalz;
kationaktive Tenside wie Cetyltrimethylammoniumchlorid.

Als weitere Hilfsstoffe seien genannt:

Viskositätserhöhende und die Emulsion stabilisierende Stoffe wie Carboxymethylcellulose, Methylcellulose und andere Cellulose- und Stärke-Derivate, Polyacrylate, Alginate, Gelatine, Gummi-arabicum, Polyvinylpyrrolidon, Polyvinylalkohol, Copolymere aus Methylvinylether und Maleinsäureanhydrid, Polyethylenglykole, Wachse, kolloidale Kieselsäure oder Gemische der aufgeführten Stoffe.

Suspensionen können oral oder als Injektion angewendet werden. Sie werden hergestellt, indem man den Wirkstoff in einer Trägerflüssigkeit gegebenenfalls unter Zusatz weiterer Hilfsstoffe wie Netzmittel, Farbstoffe, Konservierungsstoffe, Antioxidantien, Lichtschutzmittel suspendiert. Geeignet sind auch Suspensionen von Ionentauschern, die mit dem Wirkstoff beladen sind.

Als Trägerflüssigkeiten seien alle homogenen Lösungsmittel und Lösungsmittelgemische genannt.

Als Netzmittel (Dispergiermittel) seien die weiter oben angegebenen Tenside genannt.

Als weitere Hilfsstoffe seien die weiter oben angegebenen genannt.

Halbfeste Zubereitungen können oral verabreicht werden. Sie unterscheiden sich von den oben beschriebenen Suspensionen und Emulsionen nur durch ihre höhere Viskosität.

Zur Herstellung fester Zubereitungen wird der Wirkstoff mit geeigneten Trägerstoffen gegebenenfalls unter Zusatz von Hilfsstoffen vermischt und in die gewünschte Form gebracht. Als Trägerstoffe seien genannt alle physiologisch verträglichen festen Inertstoffe. Als solche dienen anorganische und organische Stoffe. Anorganische Stoffe sind z.B. Kochsalz, Carbonate wie Calciumcarbonat, Hydrogencarbonate, Aluminiumoxide, Kieselsäuren, Tonerden, gefälltes oder kolloidales Siliciumdioxid, Phosphate.

Organische Stoffe sind z.B. Zucker, Zellulose, Nahrungs- und Futtermittel wie Milchpulver, Tiermehle, Getreidemehle und -schrote, Stärken.

Hilfsstoffe sind Konservierungsstoffe, Antioxidantien, Farbstoffe, die bereits weiter oben aufgeführt worden sind.

Weitere geeignete Hilfsstoffe sind Schmier- und Gleitmittel wie z.B. Magnesiumstearat, Stearinsäure, Talkum, Bentonite, zerfallsfördernde Substanzen wie Stärke oder quervernetztes Polyvinylpyrrolidon, Bindemittel wie z.B. Stärke, Gelatine oder lineares Polyvinylpyrrolidon sowie Trockenbindemittel wie mikrokristalline Cellulose.

Die erfindungsgemäß verwendeten Chinolone können in Kombination mit anderen Wirkstoffen eingesetzt werden, und zwar gemeinsam in einer Zubereitung oder in verschiedenen Zubereitungen die gegebenenfalls auch zeitlich versetzt verabreicht werden können.

Besonders hervorgehoben seien Kombinationen mit:
- Anderen Antibiotika/Chemotherapeutika wie Betalactame (z.B. Amoxicillin ggf. in Kombination mit Clavulansäure), Cephalosporine (z.B. Cefaperazon); Makrolide (z.B. Erythromycin); Aminoglykoside (z.B. Gentamicin); Tetracycline (z.B. Doxycyclin) und Sulfonamide.
- oralen Antiseptika (z.B. Chlorhexidin)
- Cortikoide (SAIDs = *steroidal antiinflammatory drugs),* z.B. Prednisolon, Methylprednisolon, Triamcinolon, Dexamethason, Betamethason, Flumethason.
- Nicht-steroidale Antiphlogistika (NSAIDs) z.B. Phenylbutazon, Naproxen, Ketoprofen, Carprofen, Vedaprofen, Meclofenaminsäure, Flunixin, Tolfenaminsäure, Meloxicam.

Die oben aufgeführten anderen Antibiotika/Chemotherapeutika eignen sich insbesondere auch zur zeitlich versetzten Kombination in einer sequentiellen Therapie.

Anwendungsfertige Zubereitungen enthalten die Wirkstoffe in Konzentrationen von jeweils 10 ppm bis 20 Gewichtsprozent, bevorzugt von 0,1 bis 10 Gewichtsprozent.

Zubereitungen, die vor Anwendung verdünnt werden, enthalten die Wirkstoffe jeweils in Konzentrationen von 0,5 bis 90 Gewichtsprozent, bevorzugt von 1 bis 50 Gewichtsprozent.

Im Allgemeinen hat es sich als vorteilhaft erwiesen, Mengen von etwa 0,5 bis etwa 50 mg, bevorzugt 1 bis 20 mg, Wirkstoff je kg Körpergewicht pro Tag zur Erzielung wirksamer Ergebnisse zu verabreichen.

In der Mischung mit anderen Wirkstoffen liegen die erfindungsgemäß verwendeten Chinolon-Antibiotika im Verhältnis 1 zu 0,1 - 10 bis 1 zu 1 - 10 vor. Bevorzugt ist das Verhältnis 1 zu 5.

Die Wirkstoffe können auch zusammen mit dem Futter oder Trinkwasser der Tiere verabreicht werden.

Futter- und Nahrungsmittel enthalten 0,01 bis 250 ppm, vorzugsweise 0,5 bis 100 ppm des Wirkstoffs in Kombination mit einem geeigneten essbaren Material.

Ein solches Futter- und Nahrungsmittel kann sowohl für Heilzwecke als auch für prophylaktische Zwecke verwendet werden.

Die Herstellung eines solchen Futter- oder Nahrungsmittels erfolgt durch Mischen eines Konzentrats oder einer Vormischung, die 0,5 bis 30 %, vorzugsweise 1 bis 20 Gew.-% eines Wirkstoffs in Mischung mit einem essbaren organischen oder anorganischen Träger enthält, mit üblichen Futtermitteln. Essbare Träger sind z.B. Maismehl oder Mais- und Sojabohnenmehl oder Mineralsalze, die vorzugsweise eine geringe Menge eines essbaren Staubverhütungsöls, z.B. Maisöl oder Sojaöl, enthalten. Die hierbei erhaltene Vormischung kann dann dem vollständigen Futtermittel vor seiner Verfütterung an die Tiere zugesetzt werden.

### Beispiele

### Beispiel A: Ergebnisse von in vitro Studien

Geometrisches Mittel der MHK-Werte (Geometric Mean MIC; GMIC) aus Hunden und Katzen isolierter anaerober Bakterien im Vergleich zum Standard Metronidazol (in µg/ml). Ergebnisse aus zwei *in vitro* Studien:

| Bakteriengattung | N | GMIC Pradofloxacin | GMIC Metronidazol |
|---|---|---|---|
| *Bacteroides* | 37 | 0.4 | 0.7 |
| *Fusobacterium* | 11 | 0.5 | 1 |
| *Peptostreptococcus* | 4 | 0.4 | 0.8 |
| *Porphyromonas* | 8 | 0.1 | 0.3 |
| *Prevotella* | 25 | 0.2 | 0.4 |

### Beispiel B: Klinische Studie in der Indikation Parodontitis

Die Studie wurde an 16 weiblichen Beaglehunden durchgeführt. Pradofloxacin wurde in einer Dosis von 3 mg/kg einmal täglich über einen Zeitraum von sieben Tagen verabreicht.

Die Behandlung mit Pradofloxacin führte zu einer signifikanten Verringerung (p = 0.02) der Tiefe der Zahnfleischtaschen (periodontal pocket depth, loss of attachment).

Am Ende der Behandlung mit Pradofloxacin war die Gesamtzahl der anaeroben Bakterien in den Zahnfleischtaschen signifikant reduziert (p < 0.0001). Dies galt auch noch drei Wochen nach der Behandlung an Tag 28 (p = 0.0007).

### Beispiel C: In vitro Studie

In dieser Studie wurden je zwei Stämme von an der Parodontitis ursächlich beteiligten Bakterien isoliert und ihre Empfindlichkeit gegenüber Pradofloxacin *in vitro* bestimmt (MIC in µg/ml). Die Ergebnisse stellen sich wie folgt dar:
*Actinobacillus actinomycetemcomitans:* MIC < 0.25 (beide Stämme)
*Eikenella corrodens:* MIC < 0.25 (beide Stämme)
*Capnocytophaga ochracea:* MIC < 0.25 (beide Stämme)
*Porphyromonas gingivalis:* MIC < 0.25 (beide Stämme)
*Porphyromonas canoris:* MIC < 0.25 (beide Stämme)
*Porphyromonas cangingivalis:* MIC = 0.5 und MIC = 1
*Porphyromonas cansulci:* MIC < 0.25 (beide Stämme)
*Prevotella intermedia:* MIC < 0.25 (beide Stämme)
*Fusobacterium nucleatum:* MIC = 0.5 und MIC = 1
*Campylobacter rectus:* MIC < 0.25 (beide Stämme)

## Patentansprüche

1. Verwendung von
8-Cyano-Chinolon-Antibiotika
zur Herstellung von Arzneimitteln zur systemischen Behandlung von bakteriellen Erkrankungen der Mundhöhle.

2. Verwendung gemäß Anspruch 1 von 8-Cyano-Chinolon-Antibiotika der Formel (I): 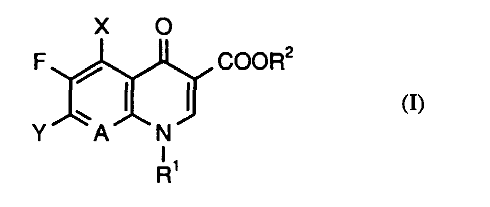 in welcher
X für Wasserstoff, Halogen, C₁₋₄-Alkyl, C₁₋₄-Alkoxy, NH₂ steht,
Y für Reste der Strukturen 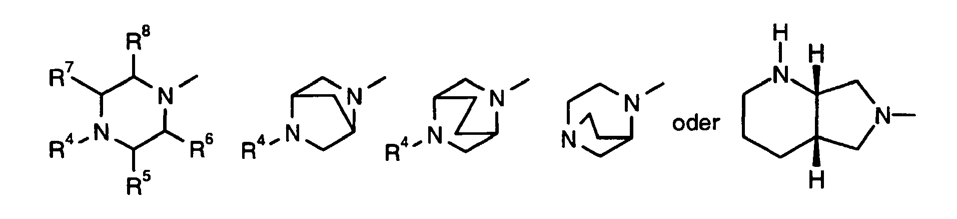
steht, worin
R⁴ für gegebenenfalls durch Hydroxy oder Methoxy substituiertes geradkettiges oder verzweigtes C₁-C₄-Alkyl, Cyclopropyl, Acyl mit 1 bis 3 C-Atomen steht,
R⁵ für Wasserstoff, Methyl, Phenyl, Thienyl oder Pyridyl steht,
R⁶ für Wasserstoff oder C₁₋₄-Alkyl steht,
R⁷ für Wasserstoff oder C₁₋₄-Alkyl steht,
R⁸ für Wasserstoff oder C₁₋₄-Alkyl steht,
sowie
R¹ für einen Alkylrest mit 1 bis 3 Kohlenstoffatomen, Cyclopropyl, 2-Fluorethyl, Methoxy, 4-Fluorphenyl, 2,4-Difluorphenyl oder Methylamine steht,
R² für Wasserstoff oder gegebenenfalls durch Methoxy oder 2-Methoxyethoxy substituiertes Alkyl mit 1 bis 6 Kohlenstoffatomen sowie Cyclohexyl, Benzyl, 2-Oxopropyl, Phenacyl, Ethoxycarbonylmethyl, Pivaloyloxymethyl steht,
A für =C(CN) steht,
und deren pharmazeutisch verwendbare Salze und Hydrate.

3. Verwendung gemäß Anspruch 1, wobei das 8-Cyano-Chinolon-Antibiotikum Pradofloxacin ist.

4. Verwendung gemäß Anspruch 1 zur Behandlung von Gingivitis, Stomatitis, Parodontitis und/oder Abszessen der Mundhöhle.

5. Verwendung gemäß Anspruch 1, wobei die Erkrankungen im wesentlichen verursacht werden durch Bakterien der Gruppe *Porphyromonas* spp., *Prevotella* spp., *Bacteroides* spp., *Actinobacillus actinomycetemcomitans, Fusobacterium* spp., *Peptostreptococcus* spp., *Eikenella corrodens, Capnocytophaga ochracea, Campylobacter rectus.*

## Claims

1. Use of
8-cyanoquinolone antibiotics for preparing medicaments for the systemic treatment of bacterial infections of the oral cavity.

2. Use according to Claim 1 of 8-cyanoquinolone antibiotics of the formula (I): 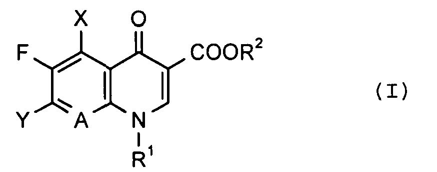 in which
X represents hydrogen, halogen, C₁₋₄-alkyl, C₁₋₄-alkoxy, NH₂,
Y represents radicals of the structure 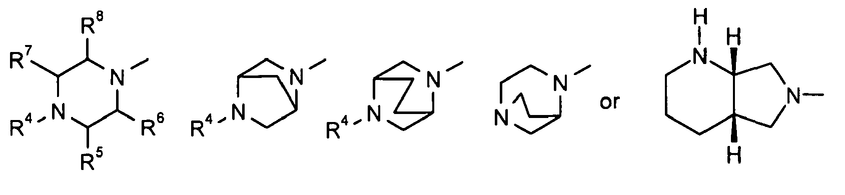
in which
R⁴ represents optionally hydroxyl- or methoxy-substituted straight-chain or branched C₁₋₄-alkyl, cyclopropyl, acyl having 1 to 3 carbon atoms,
R⁵ represents hydrogen, methyl, phenyl, thienyl or pyridyl,
R⁶ represents hydrogen or C₁₋₄-alkyl,
R⁷ represents hydrogen or C₁₋₄-alkyl,
R⁸ represents hydrogen or C₁₋₄-alkyl,
and
R¹ represents an alkyl radical having 1 to 3 carbon atoms, cyclopropyl, 2-fluoroethyl, methoxy, 4-fluorophenyl, 2,4-difluorophenyl or methylamino,
R² represents hydrogen or optionally methoxy- or 2-methoxyethoxy-substituted alkyl having 1 to 6 carbon atoms and also cyclohexyl, benzyl, 2-oxopropyl, phenacyl, ethoxycarbonylmethyl, pivaloyloxymethyl,
A represents =C(CN),
and their pharmaceutically acceptable salts and hydrates.

3. Use according to Claim 1 where the 8-cyanoquinolone antibiotic is Pradofloxacin.

4. Use according to Claim 1 for treating gingivitis, stomatitis, parodontitis and/or abscesses of the oral cavity.

5. Use according to Claim 1 where the disorders are mainly caused by bacteria of the group consisting of *Porphyromonas* spp., *Prevotella* spp., *Bacteroides* spp., *Actinobacillus actinomycetemcomitans, Fusobacterium* spp., *Peptostreptococcus* spp., *Eikenella corrodens, Capnocytophaga ochracea, Campylobacter rectus.*

## Revendications

1. Utilisation d'antibiotiques de type 8-cyano-quinolone pour la fabrication de médicaments destinés au traitement systémique de maladies bactériennes de la cavité buccale.

2. Utilisation selon la revendication 1 d'antibiotiques de type 8-cyano-quinolone de formule (I) : 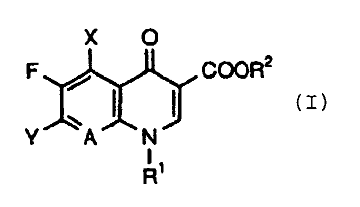 dans laquelle
x représente un atome d'hydrogène ou d'halogène, un groupe alkyle en C₁-C₄, alcoxy en C₁-C₄, NH₂,
Y représente des radicaux de structures 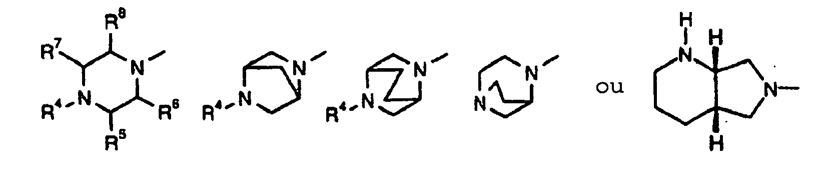
dans lesquelles
R⁴ représente un groupe alkyle en C₁-C₄ à chaîne droite ou ramifiée, cyclopropyle, acyle ayant de 1 à 3 atomes de carbone, éventuellement substitué par hydroxy ou méthoxy,
R⁵ représente un atome d'hydrogène, un groupe méthyle, phényle, thiényle ou pyridyle,
R⁶ représente un atome d'hydrogène ou un groupe alkyle en C₁-C₄,
R⁷ représente un atome d'hydrogène ou un groupe alkyle en C₁-C₄,
R⁸ représente un atome d'hydrogène ou un groupe alkyle en C₁-C₄,
ainsi que
R¹ représente un radical alkyle ayant de 1 à 3 atomes de carbone, cyclopropyle, 2-fluoroéthyle, méthoxy, 4-fluorophényle, 2,4-difluorophényle ou méthylamino,
R² représente un atome d'hydrogène ou un groupe alkyle ayant de 1 à 6 atomes de carbone, éventuellement substitué par méthoxy ou 2-méthoxyéthoxy, ainsi qu'un groupe cyclohexyle, benzyle, 2-oxopropyle, phénacyle, éthoxycarbonylméthyle, pivaloyloxyméthyle,
A représente =C(CN),
et de leurs sels et hydrates pharmaceutiquement utilisables.

3. Utilisation selon la revendication 1, dans laquelle l'antibiotique de type 8-cyano-quinolone est la pradofloxacine.

4. Utilisation selon la revendication 1, pour le traitement de la gingivite, de la stomatite, de la parodontose et/ou d'abcès de la cavité buccale.

5. Utilisation selon la revendication 1, les maladies étant essentiellement causées par des bactéries du groupe consistant en *Porphyromonas* spp., *Prevotella* spp., *Bacteroides* spp., *Actinobacillus actinomycetemcomitans, Fusobacterium* spp., *Peptostreptococcus* spp., *Eikenella corrodens, Capnocytophaga ochracea, Campylobacter rectus.*
